# EUROPEAN PATENT SPECIFICATION

(11) **EP 3 047 864 B1**
(45) Date of publication and mention of the grant of the patent: **07.03.2018**
(21) Application number: 15152110.1
(22) Date of filing: 22.01.2015
(51) Int. Cl.: A61M 1/02, A61M 1/36, G01N 33/49, B01L 3/00

(54) **Compositions and methods for separation of platelet rich plasma**
Zusammensetzungen und Verfahren zur Trennung von blutplättchenreichem Plasma
Compositions et procédés de séparation de plasma riche en plaquettes

(43) Date of publication of application: 27.07.2016
(73) Proprietor: THE REGENTS OF THE UNIVERSITY OF CALIFORNIA, Oakland CA 94607-5200 (US); University of Southern California, Los Angeles, CA 90089-5013 (US)
(72) Inventor: Emerson, Jane F., Irvine, CA 92603 (US)
(74) Representative: Dennemeyer & Associates S.A.

(56) References cited:
- WO-A1-2014/120797
- US-A1- 2014 113 795
- US-B2- 7 674 388

## Description

### Field of the Invention

The field of the invention is separation technologies.

### Background

The background description includes information that may be useful in understanding the present invention. It is not an admission that any of the information provided herein is prior art or relevant to the presently claimed invention, or that any publication specifically or implicitly referenced is prior art.

Platelet-Rich Plasma (PRP) therapy has been used for bone repair and regeneration, plastic surgery, oral surgery applications and eye treatment, and is receiving increased attention and recognition for its effectiveness in treating sports injuries, nerve injuries, tendonitis and osteoarthritis. PRP can be viewed as a concentrated source of platelets that can be delivered to the site of an injury, and activated to allow for rapid growth factor release and stimulation of bone or soft tissue recovery. Early unintentional activation of platelets should be avoided as many growth factors have short half-lives and greater effectiveness can result if they are activated at or just prior to use (e.g., injection).

Some effort has been set forth in preparing or separating PRP using centrifugation methods. Examples include U.S. Patent Nos. 6,596,180, 6,610,002, 6,827,863, 6,899,813, U.S. Patent Application Publication Nos. 2014/0113795 and 2009/0294383, and International Patent Application Publication No. WO 02/081007. Unfortunately, known methods suffer at least one of several disadvantages, including high platelet activation, inability to remove all or substantially all of the PRP without risking aliquoting a buffy coat or gel with the PRP, or inability to obtain homogeneity of the PRP and the requirement for sterile open tube processing.

*"*Platelet Separation From Whole Blood in an Aqueous Two-Phase System With WaterSoluble Polymers" by Sumida et al. published in J Pharmacol Sci 101, 91-97 (2006) recognized a problem of high platelet activation using conventional centrifugation methods, and developed a method to separate platelets without centrifugation. More specifically, various polymers were mixed with whole blood containing citrate dextrose and observed blood cell separation and platelet activation. Unfortunately, *Sumida* found that where polymers separated platelets more efficiently, more platelets were activated. Conversely, where decreased activation of platelets was achieved, there was a poor platelet yield. In addition, because of sedimentation protocols, platelets are typically not homogenously distributed in PRP fractions, and quantification is therefore difficult.

Thus, there is still a need for improved methods of preparing PRP.

Additionally, prior separation efforts apparently require aliquoting a PRP (or other fraction of a fluid) from the collection tube in which it is prepared or separated for transfer into a separate storage container for commercial uses. The aliquoting step can have one or more undesired effects, including for example, not aliquoting all of the PRP or other desired fraction, aliquoting unwanted fractions or materials, or contamination.

Thus, there is still a need for a method or device that enables the use of a desired fraction of a fluid without the need to aliquot the desired fraction from a collection tube in which it is obtained or separated for placement in a separate storage container.

### Summary of The Invention

The invention is defined by the appended claims. The inventive subject matter provides apparatuses, systems and methods in which a platelet-rich plasma (PRP) fraction having a desired concentration of functioning platelets is separated from a sample of whole blood. The separation is achieved using a polymerizable separator substance that is flowable with whole blood and curable to form a solid barrier.

As used in the description herein and throughout the claims that follow, the meaning of "a," "an," and "the" includes plural reference unless the context clearly dictates otherwise. Also, as used in the description herein, the meaning of "in" includes "in" and "on" unless the context clearly dictates otherwise.

As used herein, a PRP fraction comprises at least 150% of the platelet concentration in the sample of whole blood, less than 20% of the white blood cells from the sample of whole blood it is obtained from, and less than 20% of the red blood cells from the sample of whole blood it is obtained from.

The polymerizable separator substance can be included in a collection tube with the sample of whole blood that is centrifuged until the polymerizable separator substance settles between the PRP fraction and a non-PRP fraction of the sample of whole blood. Since the polymerizable separator substance (prior to curing) comprises smaller building blocks (e.g., oligomers), substantially less friction and shear forces result between the platelets and the separator substance materials, resulting in lower activation of platelets.

Once the polymerizable separator substance settles between the PRP and non-PRP fractions, the polymerizable separator substance can be hardened to form a solid barrier, wherein the polymerizable separator substance of the solid barrier has an average molecular weight that is higher than the average molecular weight of the polymerizable separator substance when flowable, and wherein the polymerization does not have a substantially adverse affect platelet viability. The solid barrier will preferably be stationary with respect to the collection tube and impermeable to a degree that prevents mixing of the PRP fraction and the non-PRP fraction upon agitation. This will advantageously allow a user to homogenize the PRP fraction and entirely remove it from the collection tube without remixing the PRP and non-PRP fractions.

The inventive subject matter also provides apparatuses, systems and methods in which a PRP fraction or any other fraction of any other fluid can be used without the need to aliquot the desired fraction from the collection tube in which it is obtained or separated for placement in a separate storage container.

For example, an adapter having a needle that is fluidly coupled to a fluid dispenser (e.g., an eye drop dispenser) is contemplated. The needle is sized and dimensioned to pierce at least a portion of the collection tube cap and to contact the desired fraction. The fluid dispenser will be outside of the collection tube when the needle is disposed at least partially within the tube, and includes or is coupled to a mechanism that allows sterile, controlled, drop-wise dispensing of the fluid fraction.

Various objects, features, aspects and advantages of the inventive subject matter will become more apparent from the following detailed description of preferred embodiments, along with the accompanying drawing figures in which like numerals represent like components.

### Brief Description of the Drawing

**Figure 1** illustrates a collection tube being used in accordance with a method of the inventive subject matter for separating a platelet rich plasma fraction of whole blood.
**Figure 2** is a schematic illustration of a method of separating a platelet rich plasma fraction from a sample of whole blood.
**Figure 3** shows the "Cts" (cycle threshold) of the control, the tube with LAI, and the tube with LAIT.
**Figure 4** shows the non-reproducability of cfDNA counts taken from unmixed plasma.
**Figure 5** shows aggregation of a control, LAI and LAIE to Ristocetin.
**Figure 6** shows aggregation of a control, LAI and LAIE to Collagen.
**Figure 7** is a schematic illustration of another method of separating a platelet rich plasma fraction from a sample of whole blood.
**Figure 8** is a schematic illustration of yet another method of separating a platelet rich plasma fraction from a sample of whole blood.
**Figure 9A** illustrates an embodiment of an adapter of the inventive subject matter.
**Figure 9B** illustrates an embodiment of a collection tube that can be coupled with the adapter of Figure 5A.
**Figure 9C** is a top view of a collection tube cap.

### Detailed Description

The following discussion provides many example embodiments of the inventive subject matter.

One should appreciate that the disclosed techniques provide many advantageous technical effects including increased platelet separation in the presence of a PSS without substantial activation of the separated platelets. Additionally, the compositions and methods provided allow a user to invert a collection tube upon polymerization of the separator substance to mix the separated PRP fraction, without remixing with the separated phases or the PSS, until a substantially homogeneous distribution of platelets is obtained. Additionally, the methods and devices provided allow for commercial use of a PRP or other fraction of a fluid without removing the fraction from the collection tube in which it was prepared or separated for placement into a separate storage container.

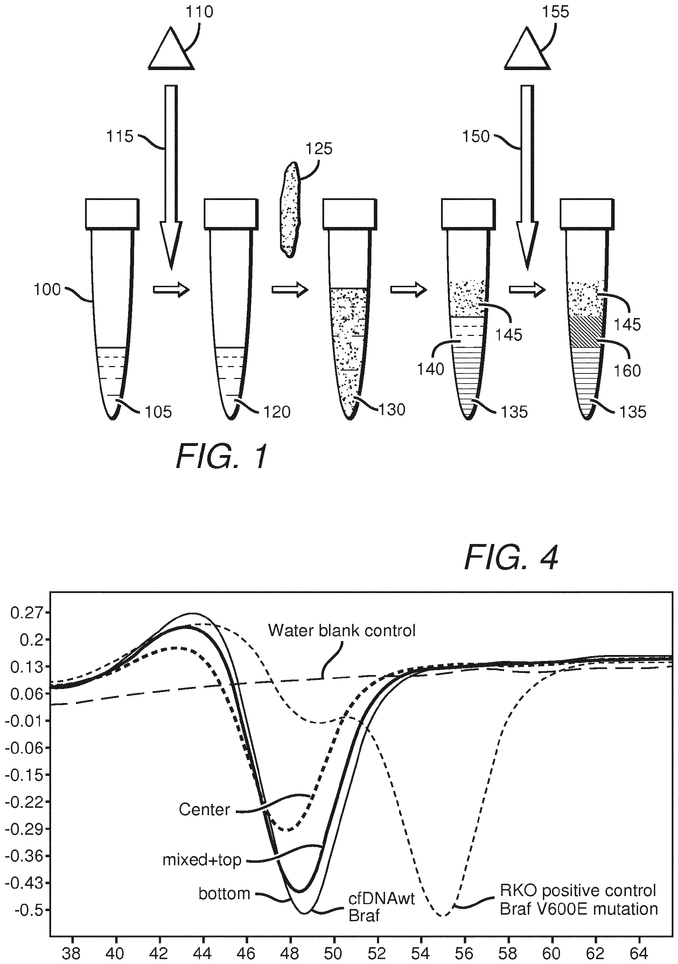

One embodiment of separating a PRP fraction in accordance with the inventive subject matter is schematically shown in **Figure 1****.** It is contemplated that any suitable collection tube could be used to perform methods of the inventive subject matter. The tube is preferably made of a rigid material (e.g., hard plastics, glass, etc.) suitable to support a vacuum within its lumen, and is of sufficient volume to hold a desirable sample of whole blood from which a desirable amount of PRP can be separated. An exemplary tube includes the BD Vacutainer® products. Although the preferred devices and methods described herein include the use of a collection tube, it is contemplated that a collection tube could be replaced with other vessels such as flasks, jars, beakers, bottles or vials.

In the embodiment of Figure 1, a collection tube 100 that contains a flowable / polymerizable separator substance 105 is exposed to a sterilizing energy source 110, which applies sterilization energy 115 (e.g., gamma radiation, e-beam radiation, heat) to the tube 100 and separator substance 105, resulting in a sterilized separation tube and sterilized (but still flowable and UV curable) separator substance 120. A sample of whole blood 125 can be added to tube 100, thereby forming a mixture 130 of the sample and sterilized separator substance.

The tube 100 and mixture 130 can then be centrifuged until the separator substance 140 component of the mixture 130 settles between a PRP fraction 145 and a non-PRP fraction 135 of the sample of whole blood 125. The PRP fraction 145 will preferably have a platelet concentration that is at least 150% the platelet concentration of the sample 125, and the non-PRP fraction 135 will preferably comprise at least 90% of the WBCs and RBCs from sample 125.

Polymerizable separator substance 140 can be polymerizable in whole or in part. Therefore, exposure of the separator substance 140 to energy 150 (e.g., UV energy) generated by a suitable energy source 155 (e.g., UV light source) will initiate radical polymerization and cause at least a portion of the separator substance 140 to form a solid, crosslinked composition 160 that acts as an impermeable barrier between the PRP fraction 145 and the non-PRP fraction 135. In some embodiments, the final thickness of the barrier (after UV curing) will be no more than 10mm, and more preferably no more than 5mm.

The polymerizable separator substance (or the polymerizable portion of a polymerizable separator substance) could be formulated to polymerize within ten minutes to a hardness of at least 1 on the Shore 00 hardness scale, more preferably a hardness of at least 10 on the Shore A hardness scale, when triggered by a suitable energy source (e.g., UV light), and form a solid with respect to a probe, a pipette, decanting or even freezing. The hardened solid barrier formed can adhere to the walls of a lumen of a tube to substantially or completely seal the PRP fraction from one or more other fractions, thereby protecting the PRP fraction from contamination due to diffusion, agitation, sample extraction, or other undesirable interaction.

Where a separator substance 140 is polymerizable only in part, it is contemplated that the non-polymerizable portion (e.g., a thixotropic gel component) can settle below the polymerizable portion such that the desired PRP fraction can be used or entirely removed from the collection tube without mixing of the PRP fraction with the non-PRP fraction or the non-polymerizable portion. The non-polymerizable portion could include, for example, off the shelf gels (e.g., BD Vacutainer® SST™, BD Vacutainer® PST™, Vacuette® blood collection tubes with gel separators, PPMA serum separator gel tubes, Polypropylene serum separator gel tubes, etc.), or any other commercially suitable gel.

It should be appreciated that a separator substance of the inventive subject matter, by virtue of the solid barrier formed, can allow a user to achieve and re-achieve substantial homogeneity of the PRP fraction by mixing or otherwise agitating the PRP fraction in the collection tube without dislodging the barrier between the PRP and non-PRP fractions. Additionally, the substantial homogeneity could be achieved without disruption of the cells, for example, without substantial activation of the platelets. Additionally, where at least a portion of the separator substance is polymerizable to form a solid, curing the polymerizable separator substance can form a polymeric network that maintains separation of the PRP fraction as efficiently as, or even better than a polymeric separator substance would.

Some contemplated polymerizable separator substances can comprise: (1) an oligomer (e.g., a combination (e.g., Ebecryl, Cytec) thereof), with (2) a photoinitiator (e.g., Additol® BDK, Additol® TPO) and (3) a stabilizer (phenothiazine). Examples of contemplated photocurable compositions include, among other things, LAI (e.g., L1A1 and phenothiazine), and LAIR. As used herein, L = an oligomer (e.g., L1 = Ebecryl 230 from Allnex, previously from Cytec Industries, Inc., etc.); A = a photoinitiator (e.g., A1 = Additol BDK); I = a stabilizer (e.g., phenothiazine, etc.). *See* **Table 1** below for more examples of components that may be included in a PSS.

**Table 1**

| **ABBREVIATION** | **NAME** |
|---|---|
| R | Gelling Agent (e.g., DBS, silica, etc.) |
| M | Monomer |
| M1 | Trimethylolpropane propoxylate triacrylate |
| L | Oligomer |
| L1 | Ebecryl 230 from Allnex |
| A | Photoinitiator |
| A1 | Additol BDK |
| I | Stabilizer |
| I1 | Phenothiazine |
| E | Antioxidant / radical scavenger (e.g., Vitamin E, BHT, BHA, carotene, bilirubin, ascorbic acid, etc.) |
| D | Density adjuster (e.g., Ebecryl 113 from Cytec, etc.) |
| T | Tempo nitroxide |

In some contemplated polymerizable separator substances, the photoinitiator is present at a concentration of less than 10wt%, less than 5wt% or even less than 2wt%, and a stabilizer is present at a concentration of less than 5wt%, less than 2wt%, less than 1wt% or even less than 0.5wt%. As a more specific, non-limiting example, a separator substance can comprise an oligomer present at a concentration of between 95 and 100% (100wt%), a photoinitator present at a concentration of between 0.8 and 1.2% (e.g., 1wt%), and a stabilizer present at a concentration of between 0.01 and 0.05% (e.g., 0.1wt%).

Contemplated photoinitiators include, among other things, Additol BDK and Additol TPO. Contemplated stabilizers include, among other things, phenothiazine. A separator substance may also include an antioxidant, especially where sterilization of the collection tube with separator substance is desired without substantial curing of the separator substance. Contemplated antioxidants include, among other things, tocopherol, butylated hydrozytoluene (BHT), butylated hydroxyanisole (BHA), carotene, bilirubin and ascorbic acid.

An exemplary polymerizable separator substance comprises an oligomer, a photoinitiator and a stabilizer, wherein the photoinitiator is present at a concentration of less than 5%, and wherein the stabilizer is present at a concentration of less than .5 wt%.

LAI (e.g., L1, A1 and phenothiazine) composes some especially preferred photocurable compositions, and can be formulated to have the desired density range of 1.00-1.09 g/cm³. Although experiments were conducted using a photocurable composition having an oligomer, it is contemplated that monomer-containing compositions may also be suitable for use in PRP preparation since many monomers and oligomers have similarly high reactivity, and many monomers and oligomers can polymerize in the presence of light.

Other examples of potentially suitable photocurable separator substances include those described in U.S. Patent Nos. 7,674,388, 7,673,758, 7,775,962, 7,971730, 7,780,861, 8,206,638, 8,282,540, 8,151,996, and 8,318077, LAIE (e.g., L1, A1, phenothiazine and tocopherol), and LAIER, wherein R = a gelling agent (e.g., DBS, silica, etc.), and E = at least one of an antioxidant and a radical scavenger (e.g., Vitamin E, butylated hydroxytoluene (BHT), butylated hydroxyanisole (BHA), carotene, bilirubin, ascorbic acid, etc.). While R and E are generally not necessary components of a photocurable composition, each can provide advantageous features to the photocurable composition for some uses. For example, a gelling agent may not be a necessary component of the photocurable composition where the separator substance also comprises a thixotropic soft gel component that is loaded into the collection tube above the photocurable composition such that no flow will result prior to use. Nonetheless, it may be desirable to have a thixotropic photocurable composition, for example, where it is desirable to have the sealant disposed in the tube without a soft gel component, or on top of the soft gel component.

A radical scavenger such as compounds having Vitamin E activity (e.g., tocopherol), while not necessary, can be included to allow the photocurable composition to be sterilized via irradiation without curing (e.g., by changing the density properties), rather than requiring heat sterilization to maintain a flowability effective to allow sedimentation between two fractions of a sample of whole blood. Where heat sterilization is desired, it can be achieved by exposing the collection tube and separator substance to a heat of at least 200 degrees Celsius, more preferably at least 225 degrees Celsius, and most preferably at least 250 degrees Celsius.

It is contemplated that an anticoagulant can be included in the collection tube, optionally as part of the separator substance, to prevent clotting of the sample of whole blood and obtain plasma containing fibrinogen and clotting factors. Contemplated anticoagulants include sodium citrate, EDTA, citrate dextrose, or any other suitable anticoagulant. It is noteworthy that even though radiation polymerization is initiated and performed, the chemistry used and especially acrylic polymerization will neither effect platelet activation or function, nor interfere with many or most other tests that can be performed on the PRP fraction.

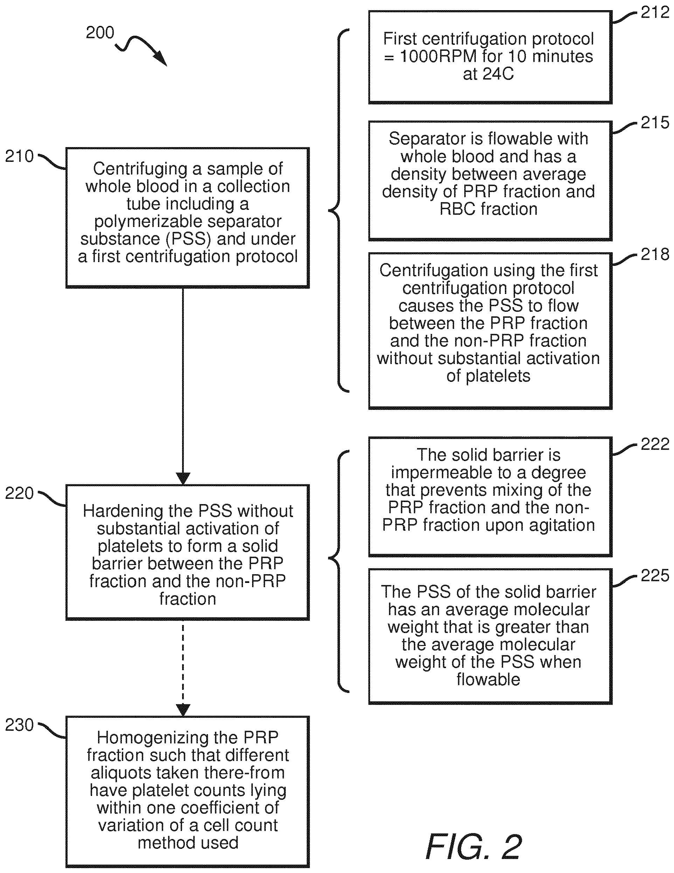

**Figure 2** illustrates a method 200 of separating a PRP fraction from a sample of whole blood in a collection tube including a polymerizable separator substance (PSS). While any suitable amount of the separator substance can be included in the tube's lumen, it is preferred that no more than about 1ml or 2 grams of the separator substance be included per 10ml volume of the tube.

Method 200 includes the step of centrifuging the sample of whole blood in the collection tube including the PSS using a centrifugation protocol as shown in step 210. Any suitable centrifugation protocol that is sufficient to cause the PSS to flow between two fractions of a sample is contemplated, including for example, centrifugation for ten minutes at 24°C and 1000 RPM as shown in step 212. Other examples of suitable centrifugation protocols could include: a centrifugation time of between one minute and thirty minutes, inclusive, at between 500 RPM and 5000 RPM, inclusive; or a centrifugation time of between five minutes and twenty minutes, inclusive, at between 700 RPM and 3600 RPM, inclusive. As is well known in the art, centrifugation conditions determine the sedimentation and sedimentation rate of the platelets. The PHOSITA will be able, without undue experimentation, to determine the appropriate applied G forces and time to achieve a desired yield of platelets above the PSS. Therefore, an increase or decrease in desired yields can be readily achieved using the above considerations.

Unless the context dictates the contrary, all ranges set forth herein should be interpreted as being inclusive of their endpoints and open-ended ranges should be interpreted to include only commercially practical values. Similarly, all lists of values should be considered as inclusive of intermediate values unless the context indicates the contrary.

In most preferred embodiments, the PSS will be flowable with the whole blood and has a density between an average density of a PRP fraction and an average density of a non-PRP fraction in accordance with step 215.

To achieve a desired initial density, it is contemplated that the density of the separator substance may be adjusted by virtue of molecular composition, or by inclusion of appropriate filler materials (e.g., silica, latex, or other inert material).

Additionally or alternatively, centrifugation under a suitable protocol can cause the PSS to flow between the PRP fraction and the non-PRP fraction without substantial activation of platelets in accordance with step 218. It is contemplated that where the PSS is used for separation of a sample of a different fluid, or for separation of a sample of whole blood into fractions other than a PRP and non-PRP fraction, it is contemplated that the PSS could have a density between an average density of a first fraction to be separated and an average density of a second fraction to be separated.

As used herein, centrifugation using a first centrifugation protocol to cause the PSS to flow between two fractions of a sample without substantial activation of platelets means that the platelets retain function to within 15%, more preferably within 10%, of what would be retained by platelets during an otherwise identical centrifugation protocol as the first centrifugation protocol where a PSS is not used (e.g., without any separator substance). Substantial activation of platelets is measurable, among other things, based on the ability of platelets in the PRP fraction, non-PRP fraction, or both, to aggregate to at least one of Ristocetin and Collagen.

Method 200 also includes step 220 of hardening the PSS without substantial activation of platelets to form a solid barrier between the PRP fraction and the non-PRP fraction. Depending on the formulation of the separator substance, it is contemplated that the mode or mechanism of polymerization can vary considerably. While the discussion herein is directed primarily towards UV energy polymerization, all known methods of polymerization are deemed suitable for use herein. For example, contemplated polymerizations include various radical or cationic polymerizations (e.g., using photolabile compounds, radical starters, etc.), condensation polymerization, esterification, amide formation and so forth. Reactive groups can include acid groups, most preferably mono- and dicarboxylic groups), conjugated diene groups, aromatic vinyl groups, and alkyl(meth)acrylate. The polymerization can advantageously be fully supported by reactive groups of the monomers or oligomers of the separator substance, but additional reagents may also be suitable, including radical starters.

The step of hardening can advantageously form a solid barrier between the PRP fraction and the non-PRP fraction that is stationary with respect to the collection tube. Additionally, the solid barrier can be impermeable to a degree that prevents mixing of the PRP fraction and the non-PRP fraction upon agitation as shown in step 222. The PSS of the solid barrier will have an average molecular weight that is greater than the average molecular weight of the PSS when flowable with the whole blood in accordance with step 225.

As used herein, hardening the polymerizable separator substance without substantial activation of platelets means that the platelets retain function to within 15%, more preferably within 10%, of what would be retained by platelets where PSS is not used and where there is no step of hardening.

The solid barrier advantageously enables a step of removing at least 90%, more preferably at least 95% and most preferably 100% of the PRP fraction from the collection tube without remixing the PRP fraction with the non-PRP fraction. This could be achieved by removing the PRP fraction as is from the collection tube (e.g., by pouring out, pipetting, etc.), or by including a saline or other fluid to the tube to assist in removing the entire PRP fraction.

Method 200 could optionally include a step of homogenizing the PRP fraction, after the step of hardening, such that different aliquots taken there-from have platelet counts lying within one coefficient of variation of a cell count method used. This can be highly beneficial in providing reproducibility and uniformity. Homogenizing the PRP fraction can be accomplished using any suitable methods, including for example, mechanical stirring, bubble stirring, turning the tube upside down or otherwise agitating, or triturating through wide mouth pipette.

### Platelet Recovery

Many in the cosmetic and health industries have found that PRP having 200-250% of a platelet concentration of a sample of blood is optimal. Collection tubes previously used (e.g., purple top BD Vacutainers with no separator substance) to recover fractions of whole blood involve centrifuging blood to result in a serum fraction supernatant, an intermediate buffy layer, and a red blood cell fraction beneath the buffy layer. Such tubes and methods make it very difficult or even impossible to remove the supernatant without removing white cells from the buffy coat. On the other hand, where a gel is used, the result is a PRP or serum fraction depending on centrifugation protocol, then an intermediate gel layer, then a buffy coat, and a bottommost RBC fraction. Again, it is very difficult if not impossible to remove the entirety of the PRP or serum fraction without aspirating the gel with the PRP or serum. And in either event, there is a significant maldistribution of platelets in the PRP fraction.

The PSS and methods of the inventive subject matter advantageously allow for platelet recovery at various concentrations (including the 200-250% range), and also allow for use of the entirety of the PRP supernatant. **Table 2** below illustrates that platelet yield using LAI or LAIR was at least as good as when using a control. Table 2 also illustrates that the same red blood cell depletion could be achieved using LAI or LAIR as when using the control.

**Table 2**

| **Blue Top (sodium citrate), Glass Tube, Fresh Blood, 30 second UV Exposure Gel Composition = L (100%); A (1.00%); I (0.10%); R (5%)** | | | | | | | | |
|---|---|---|---|---|---|---|---|---|
| | RCF (g) Time | (min) | Platelet (pre) | Platelet (post) | % Platelet Increase | RBC (Pre) | RBC (Post) | % RBC Depletion |
| control 1 | 300 | 5 | 198 | 435 | 220 | 4.19 | 0.03 | 99 |
| Control 2 | 300 | 5 | 216 | 462 | 214 | 4.71 | 0.04 | 99 |
| LAI 1 | 300 | 5 | 205 | 381 | 186 | 4.22 | 0.03 | 99 |
| LAI 2 | 300 | 5 | 194 | 392 | 202 | 4.16 | 0.03 | 99 |
| LAIR 1 | 300 | 5 | 192 | 480 | 250 | 4.18 | 0.03 | 99 |
| LAIR 2 | 300 | 5 | 201 | 469 | 233 | 4.17 | 0.06 | 99 |

| **Red Top, Glass Tube, Pooled Blood, 30 second UV Exposure Gel Composition = L (100%); A (1.00%); I (0.10%); R (5%)** | | | | | | | | |
|---|---|---|---|---|---|---|---|---|
| | RCF(g) | Time (min) | Platelet (pre) | Platelet (post) | % Platelet Increase | RBC (Pre) | RBC (Post) | % RBC Depletion |
| Control 1 | 300 | 5 | 193 | 333 | 173 | 1.96 | 0.03 | 98 |
| Control 2 | 300 | 5 | 122 | 186 | 152 | 2.05 | 0.04 | 98 |
| LAI 1 | 300 | 5 | 117 | 175 | 150 | 2.02 | 0.03 | 99 |
| LAI 2 | 300 | 5 | 141 | 267 | 189 | 2.73 | 0.03 | 99 |
| LAIR 1 | 300 | 5 | 114 | 197 | 173 | 2.11 | 0.03 | 99 |
| LAIR 2 | 300 | 5 | 121 | 195 | 161 | 2.13 | 0.06 | 97 |

| **Blue Top (sodium citrate), Glass Tube, Pooled Blood, 30 second UV Exposure Gel Composition = L (100%); A (1.00%); I (0.10%); R (5%)** | | | | | | | | |
|---|---|---|---|---|---|---|---|---|
| | RCF(g) | Time (min) | Platelet (pre) | Platelet (post) | % Platelet Increase | RBC (Pre) | RBC (Post) | % RBC Depletion |
| Control 1 | 200 | 12 | 121 | 155 | 128 | 2.20 | 0.03 | 99 |
| Control 2 | 200 | 12 | 143 | 165 | 115 | 2.08 | 0.03 | 99 |
| LAI 1 | 200 | 12 | 134 | 164 | 122 | 2.19 | 0.03 | 99 |
| LAI 2 | 200 | 12 | 138 | 164 | 119 | 2.14 | 0.03 | 99 |
| LAIR 1 | 200 | 12 | 122 | 161 | 132 | 2.18 | 0.03 | 99 |
| LAIR 2 | 200 | 12 | 122 | 169 | 139 | 2.07 | 0.03 | 99 |
| **Blue Top (sodium citrate), Glass Tube, Pooled Blood, 30 second UV Exposure Gel Composition = L (100%); A (1.00%); I (0.10%); R (5%)** | | | | | | | | |

| | RCF (g) | Time (min) | Platelet (pre) | Platelet (post) | % Platelet Increase | RBC (Pre) | RBC (Post) | % RBC Depletion |
|---|---|---|---|---|---|---|---|---|
| LAI 1 | 150 | 10 | 171 | 247 | 144 | 2.58 | 0.04 | 98 |
| LAI 2 | 150 | 10 | 177 | 211 | 119 | 2.04 | 0.05 | 98 |
| LAIR 1 | 150 | 10 | 187 | 228 | 122 | 1.8 | 0.05 | 97 |
| LAIR 2 | 150 | 10 | 198 | 248 | 125 | 1.54 | 0.05 | 97 |

| **Blue Top (sodium citrate), Glass Tube, Pooled Blood, 30 second UV Exposure Gel Composition = L (100%); A (1.00%); I (0.10%); R (5%)** | | | | | | | | |
|---|---|---|---|---|---|---|---|---|
| | RCF(g) | Time (min) | Platelet (pre) | Platelet (post) | % Platelet Increase | RBC (Pre) | RBC (Post) | % RBC Depletion |
| LAI 1 | 150 | 20 | 183 | 243 | 133 | 2.39 | 0.03 | 99 |
| LAI 2 | 150 | 20 | 142 | 139 | 98 | 1.69 | 0.02 | 99 |
| LAIR 1 | 150 | 20 | 123 | 150 | 122 | 1.62 | 0.02 | 99 |
| LAIR 2 | 150 | 20 | 128 | 146 | 114 | 1.6 | 0.02 | 99 |

**Table 3** below illustrates that platelet yield using LAI or LAIE was at least as good as when using a control. Table 2 also illustrates that the same or similar white blood cell depletion could be achieved using LAI or LAIE as when using the control.

The compositions (LAI and LAIE) tested are composed of the following:

| | LAI | LAIE |
|---|---|---|
| L | 100% | 100% |
| A | 1% | 1% |
| I | 0.10% | 0.10% |
| E | -- | 200 mM |

The manual differential of LAI was as follows: 8% neutrophils, 80 % lymphocytes, 10% monocytes, 2% eosinophils; The manual differential of LAIE was as follows: 5% neutrophils, 89% lymphocytes, 5% monocytes, 1% eosinophils.

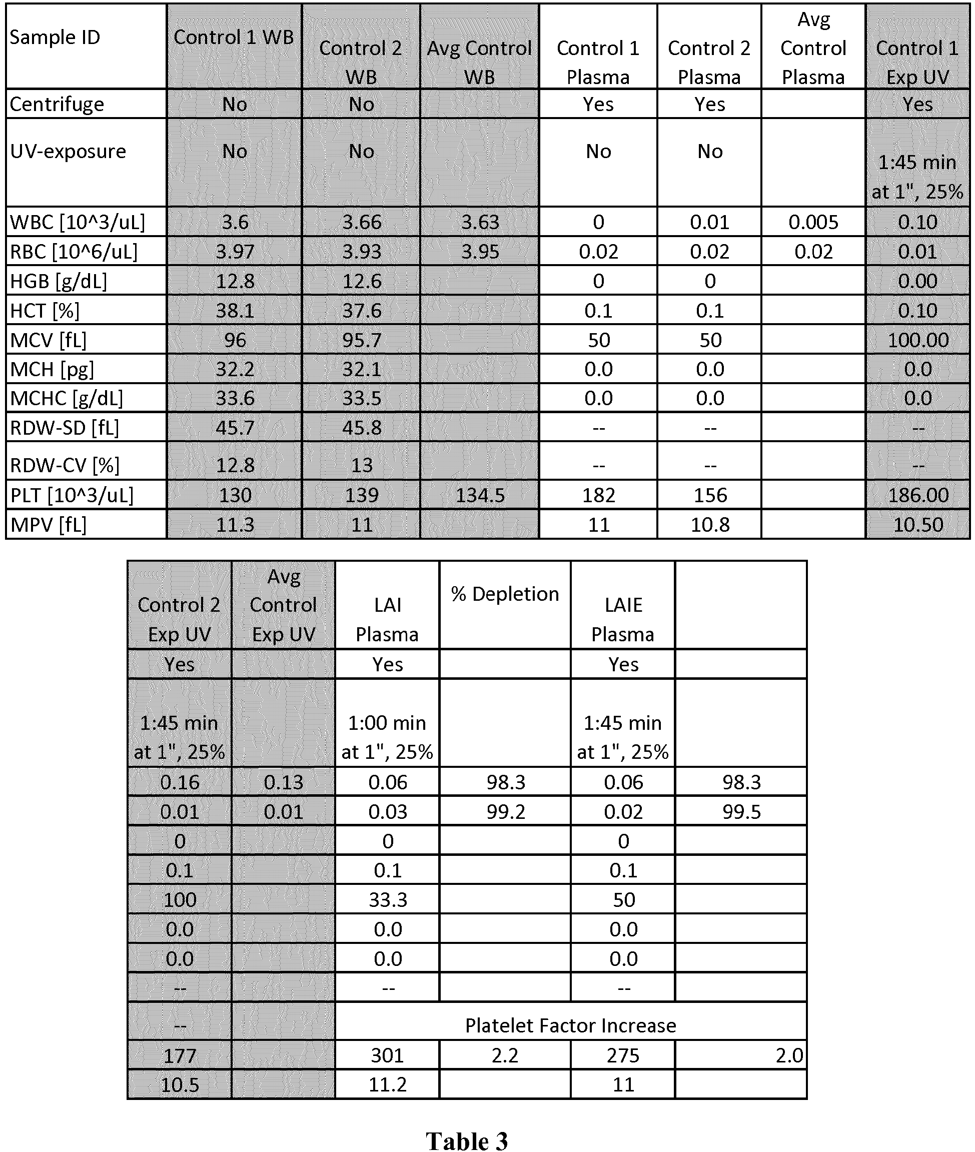

**Table 4** shows platelet yield and white blood cell depletion under a centrifugation protocol as follows: 1000 RPM for ten minutes at 24 C. There was a slight increase in platelet yield and similar white blood cell / red blood cell depletion.

The compositions (LAI and LAIE) tested are composed of the following:

| | LAI | LAIE |
|---|---|---|
| L | 100% | 100% |
| A | 1% | 1% |
| I | 0.10% | 0.10% |
| E | -- | 200 mM |

The experiments were conducted in Sodium Citrate Tubes.

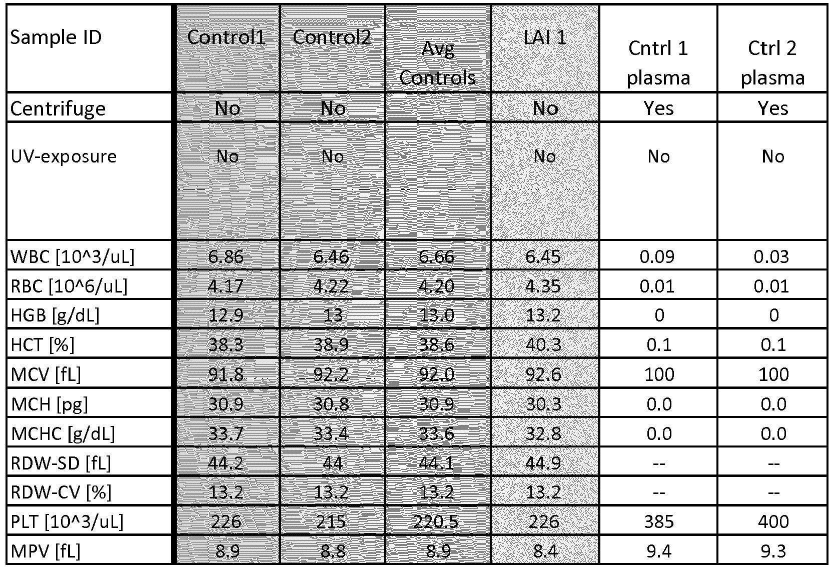

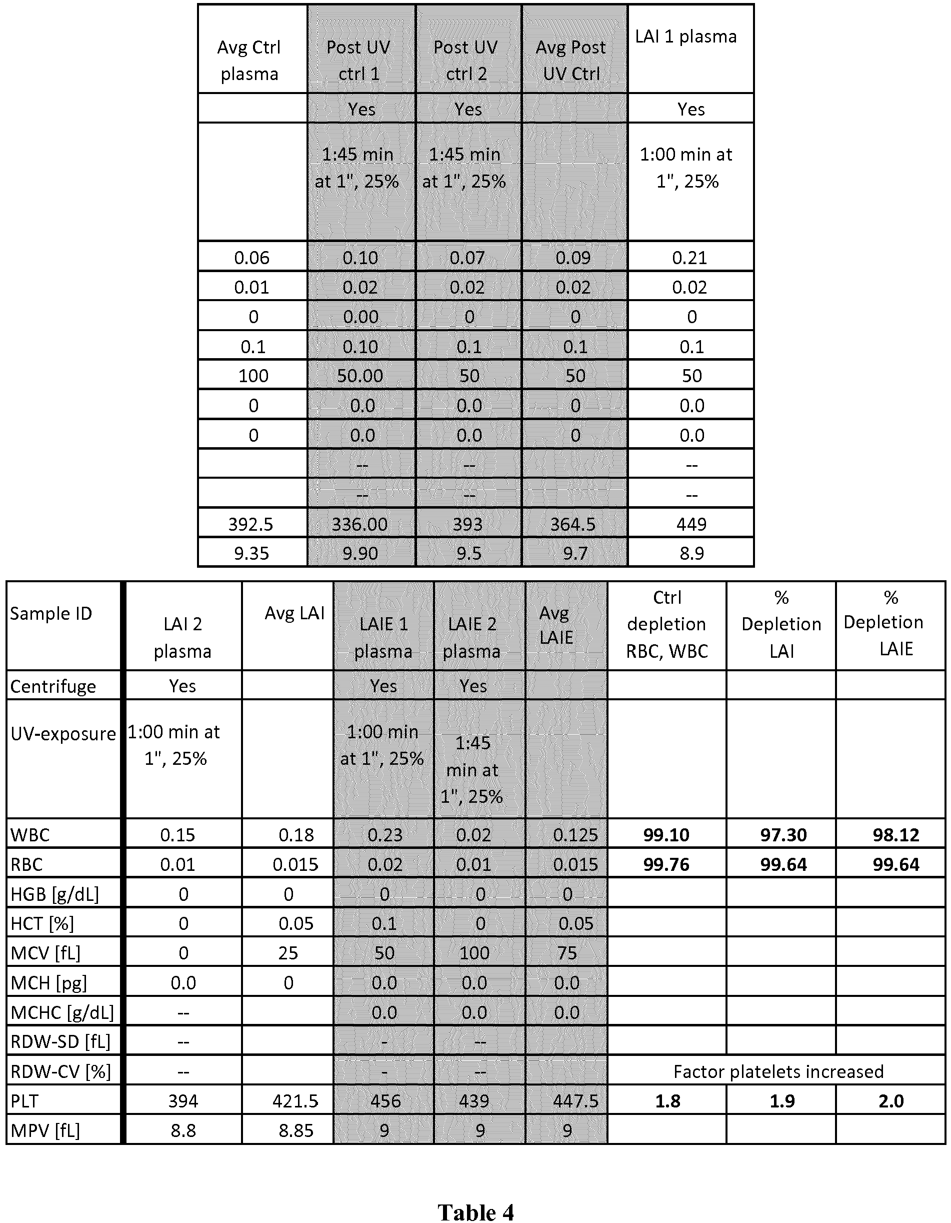

### Recovery of a Homogenous PRP Fraction

The PSS of the inventive subject matter provides an additional advantage of enabling preparation of a substantially homogenous PRP (or other) fraction. Applicant compared the recovery of cell-free DNA in 3 tube types and determined that aliquots taken from an unmixed specimen would lead to variable results. Viewed from a different perspective, if a user aliquots plasma from a non-gel tube without mixing, the aliquots will be highly variable. If a user aliquots the plasma from the tube and mix it outside of the tube, the results may be more reproducible but the recovery is worse.

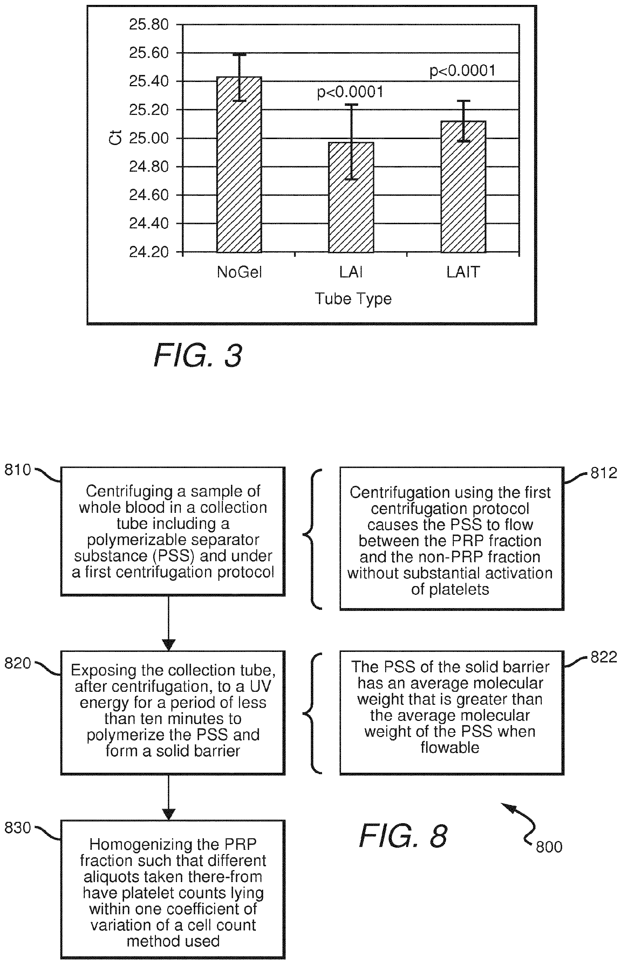

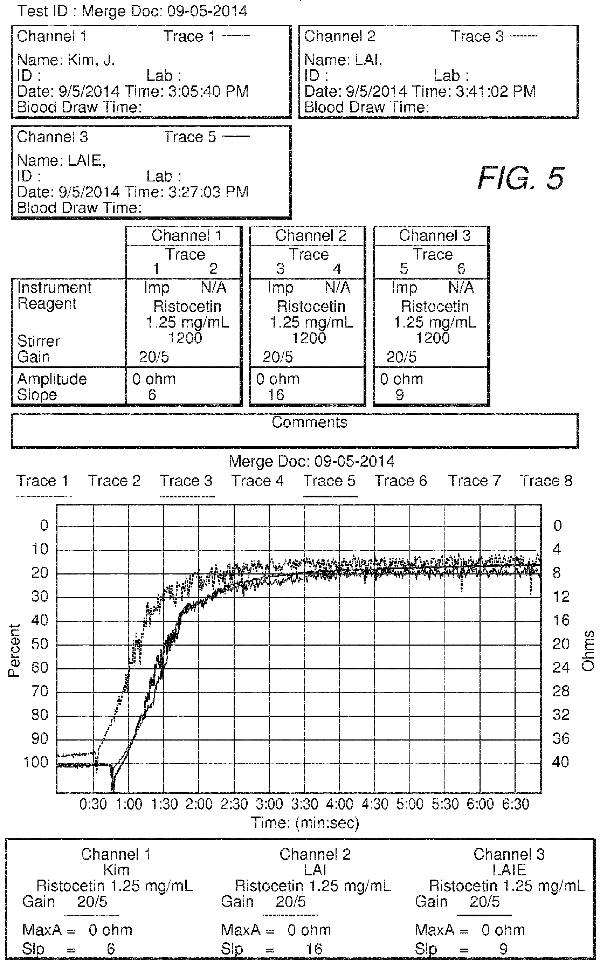

The 3 tube types tested are as follows: control ("No gel"), tube with LAI photogel and tube with LAIT photogel where L= oligomer, A=additol BDK photoinitiator, I= phenothiazine stabilizer, and T=tempo nitroxide. **Figure 3** shows the "Cts" (cycle threshold) of the control, the tube with LAI, and the tube with LAIT. The lower Ct of LAI and LAIT as compared to the control indicates better recovery as compared to the control. Figure 5 also shows that the results using LAI and LAIT were similarly reproducible as the control. N=4 for each tube type.

The protocol used is as follows: pooled blood was mixed and loaded by pipette into each of the nine tubes. The photogel tubes were preloaded with either LAI or LAIT. All tubes were centrifuged at room temperature for 10 minutes at 3000 RPM. Following centrifugation, the photogel tubes were exposed to UV light in light box for 1 minute. The plasma from each tube was withdrawn, and for tubes including the solidified barrier, all plasma was withdrawn. For the control tube with no gel, care was taken not to disrupt the cell layer which required leaving some plasma behind (approximately 100 uL). The plasma was sent to the molecular pathology lab for DNA-Braf analysis. There, the four tubes were further aliquotted into three samples each. Thus the variability shown is inclusive of the process of dividing the plasma from each tube three ways.

Plasma obtained from an EDTA tube with no gel was manually sampled at the top layer, bottom (near cells) layer, and middle portion. The plasma was mixed by vortex and sampled again ("mixed"). The melt curves shown below demonstrate that the cfDNA recovered varies with manual sampling. Cell free DNA was used as a proxy marker to platelet recovery.

In the example shown in **Figure 4**, the aliquot taken from the bottom layer of the unmixed plasma had more cfDNA than aliquot taken from a center portion or a mixed top portion (deeper trough indicates greater recovery).

Because the methods described herein include the use of a PSS that is hardened to form a solid barrier, the PRP fraction can be mixed inside the collection tube in which it is obtained or separated, which allows for homogenizing of the PRP fraction in the collection tube without exposure to an environment outside of the collection tube. It should be appreciated that the PSS allows a user to obtain a homogenous reliable count of platelets because amounts obtained from different portions of the tube can be within one coefficient of variation of a cell count method used.

### Platelet Viability

As discussed above, Applicants were able to demonstrate that similar or even improved platelet recovery could be achieved using methods and PSSs of the inventive subject matter. It is known in the art that solid barriers with long polymers, while often improving platelet recovery, interfere with platelet function. The experiments and data provided below show that Applicant's methods and PSSs allow for optimal platelet recovery while maintaining viability of the platelets.

Platelet viability was tested by observing platelet aggregation to specific agonists on the Chrono-log aggregometer. The PRP was obtained by centrifuging whole blood loaded into a control tube (BD sodium citrate tube, no gel), LAI tube (BD sodium citrate to which LAI was added), and LAIE tube (BD sodium citrate tube with LAIE added). The photogel tubes were exposed to UV light after centrifugation to solidify the barrier. The PRP was aliquotted and diluted with platelet poor plasma to a platelet concentration required by the Chrono-log standard operating procedure. Below are example curves of platelet aggregation to Ristocetin and Collagen. Collagen is a strong agonist that induces aggregation, secretion of platelet granules and thromboxane synthesis, thus it is an inclusive test for platelet viability.

As illustrated in **Figure 5**, no significant different in aggregation to Ristocetin was shown between the control, LAI and LAIE.

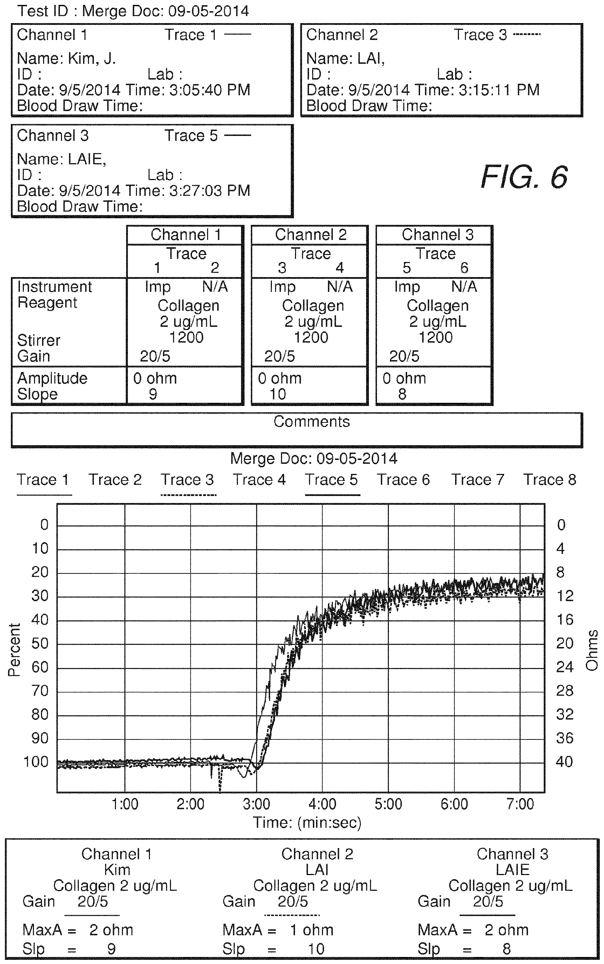

As illustrated in **Figure 6**, no appreciable difference is seen in platelet aggregation curves for platelets to Collagen from control, LAI or LAIE curves, which indicates that the LAI and LAIE do not substantially affect platelet functionality.

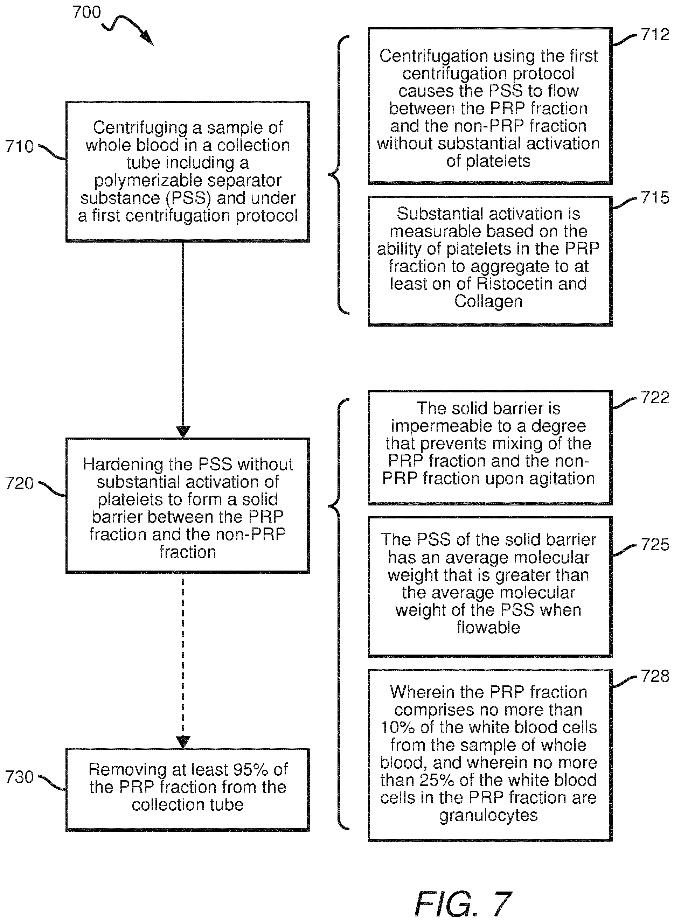

Figure 7 illustrates another method 700 of separating a PRP fraction from a sample of whole blood in a collection tube including a PSS that is flowable with whole blood and has a density between an average density of a PRP fraction and an average density of a non-PRP fraction of whole blood.

Method 700 includes the step of centrifuging the sample of whole blood in the collection tube including the PSS using a centrifugation protocol as shown in step 710. Similarly to the centrifugation step of method 200, centrifugation using a suitable centrifugation protocol will cause the PSS to flow between the PRP fraction and the non-PRP fraction without substantial activation of platelets as shown in step 712. Substantial activation is measurable based on the ability of platelets in the PRP fraction to aggregate to at least one of Ristocetin and Collagen as shown in step 715.

Method 700 further includes the step of hardening the PSS without substantial activation of platelets to form a solid barrier between the PRP fraction and the non-PRP fraction as shown in step 720. The PSS of the solid barrier will have an average molecular weight that is greater than the average molecular weight of the PSS when flowable with the whole blood (e.g., prior to the step of hardening) in accordance with step 725.

In some preferred methods, the solid barrier, after exposure to the UV energy, will be stationary with respect to the collection tube at an intermediate position below the PRP fraction and above the non-PRP fraction and be impermeable to a degree that prevents mixing of the PRP fraction and the non-PRP fraction upon agitation as shown in step 722. The solid barrier allows for removal of at least 90%, more preferably at least 95% and most preferably 100% of the PRP fraction from the collection tube. Viewed from a different perspective, method 700 includes an optional step of removing at least 95% of the PRP fraction from the collection tube in accordance with step 730.

The PRP fraction can advantageously comprise no more than 10% of white blood cells from the sample of whole blood. Additionally or alternatively, it is contemplated that no more than 25% of the white blood cells in the PRP fraction will be granulocytes.

Method 700 could also comprise homogenizing the PRP fraction in the collection tube after the step of hardening. The homogenizing step could be performed immediately after hardening, within one hour of hardening, within one day of hardening, at least two days after hardening, at least five days after hardening, or even at least one month after hardening. Homogenizing the PRP fraction results in a substantially homogenous PRP fraction such that different aliquots taken there-from have platelet counts lying within one coefficient of variation of a cell count method used.

**Figure 8** illustrates yet another method 800 of separating a PRP fraction from a sample of whole blood in a collection tube including a PSS that is flowable with whole blood and has a density between an average density of a PRP fraction and an average density of a non-PRP fraction of whole blood.

It should be appreciated that a PRP fraction could have any suitable platelet concentration that is greater than a platelet concentration of a sample of whole blood from which it is obtained or separated. For example, a PRP fraction could comprise at least 150%, at least 200%, at least 250% or even greater platelet concentration of a platelet concentration of the sample from which it is obtained. Viewed from a different perspective, a PRP fraction could comprise between a 180% and 260% platelet concentration or between a 200% and 250% platelet concentration of a platelet concentration of the sample from which it is obtained.

Method 800 includes a step of centrifuging a sample of whole blood in a collection tube including a PSS and under a first centrifugation protocol as shown in step 810. Centrifugation under the first protocol can cause the PSS to flow between the PRP fraction and the non-PRP fraction, preferably without substantial activation of platelets as shown in step 812.

Method 800 also includes a step of exposing the collection tube, after centrifugation, to a UV energy for a period of less than ten minutes to polymerize the PSS and form a solid barrier as shown in step 820. The PSS of the solid barrier will have an average molecular weight that is greater than the average molecular weight of the PSS when flowable with the sample of whole blood as shown in step 822.

Method 800 further includes a step of homogenizing the PRP fraction in the presence of the solid barrier to form a substantially homogenous PRP fraction such that different aliquots taken there-from have platelet counts lying within one coefficient of variation of a cell count method used.

The methods described herein advantageously allow a user to separate fractions of different densities of a sample (e.g., blood) included in a collection tube with a PSS. The solid barrier allows for both homogeneity and reproducibility, and applicant has discovered that further advantages could be achieved by providing an adapter such that the tubes in which the fractions are separated never need to be opened.

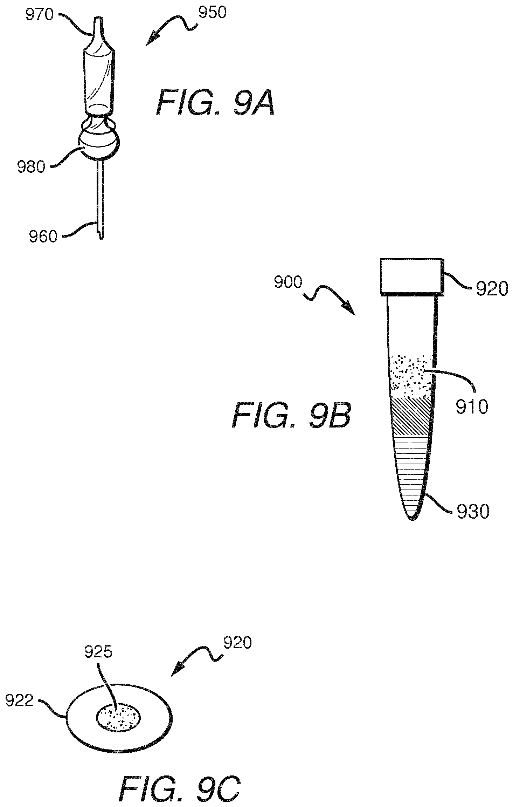

**Figure 9A-9C** illustrates a collection device 900 and an adapter 950 that can be used with the collection device to allow for closed processing while maintaining sterility through multiple uses of a fluid contained therein, resulting in a lower risk of contamination. It should be appreciated that adapter 950 could be used not only with PRP applications, but with any other fluid (e.g., serum separated from a sample of whole blood, plasma separated from a sample of whole blood, etc.) that is processed in a collection device (e.g., a collection tube or any other suitable container).

Collection device 900 includes a tube portion 930 and a tube-cap portion 920 that cooperate to block or seal a fluid 910 (here a PRP fraction), and optionally a solidified PSS and non-PRP fraction, from an environment outside of the collection tube. Tube-cap portion 920 includes a hard material 922 (e.g., a plastic) that surrounds a rubber or other material 925 that is penetrable by needle 960 of adapter 950.

Needle 960 is sized and dimensioned to pierce at least a portion of tube-cap portion 920 and contact fluid 910 within in the tube. Needle 960 is coupled to a fluid dispenser 970 that is configured as an eye dropper and positioned outside of the collection device when the needle is at least partially in the tube. Fluid dispenser 970 comprises or is coupled to a mechanism 980 (e.g., bulb, valve, etc.) that allows sterile, controlled, drop-wise dispensing of the fluid from the collection device.

As used herein, and unless the context dictates otherwise, the term "coupled to" is intended to include both direct coupling (in which two elements that are coupled to each other contact each other) and indirect coupling (in which at least one additional element is located between the two elements). Therefore, the terms "coupled to" and "coupled with" are used synonymously.

A fluid dispenser that is configured as an eye dropper can comprise a small tube and a vacuum bulb. The eye dropper can be configured to draw up fluid 910 from a first end (e.g., needle 960), and release or dispense the fluid 910 from a second end (e.g., tip of fluid dispenser 970) a single drop at a time. In some embodiments, saline or other fluid(s) can be injected or otherwise placed in the collection device with fluid 910 prior to piercing tube-cap 920 with needle 960. The PRP, with or without saline, could be used to treat or prevent dry eye syndrome or other eye conditions. It should be appreciated that an eye dropper is a device that is configured to contain a fluid, and dispense the fluid directly to an eye of a user in a controlled, drop-wise manner.

An alternative example of a contemplated fluid dispenser is a needle, which can be coupled to a syringe for injectable applications.

It should be apparent to those skilled in the art that many more modifications besides those already described are possible without departing from the inventive concepts herein. The inventive subject matter, therefore, is not to be restricted except in the scope of the appended claims. Moreover, in interpreting both the specification and the claims, all terms should be interpreted in the broadest possible manner consistent with the context. In particular, the terms "comprises" and "comprising" should be interpreted as referring to elements, components, or steps in a non-exclusive manner, indicating that the referenced elements, components, or steps may be present, or utilized, or combined with other elements, components, or steps that are not expressly referenced. Where the specification claims refers to at least one of something selected from the group consisting of A, B, C .... and N, the text should be interpreted as requiring only one element from the group, not A plus N, or B plus N, etc.

## Claims

1. A method of separating a PRP fraction from a sample of whole blood in a collection tube including a polymerizable separator substance, wherein the polymerizable separator substance is flowable with the whole blood, has a first average molecular weight, and has a density between an average density of a PRP fraction and an average density of a non-PRP fraction, wherein the separator substance comprises an oligomer, a photoinitiator and a stabilizer, the method comprising:
centrifuging the sample of whole blood in the collection tube including the polymerizable separator substance, for a period of between one and thirty minutes at between 500 and 5000 RPM;
wherein centrifuging causes the polymerizable separator substance to flow between the PRP fraction and the non-PRP fraction without substantial activation of platelets in the PRP fraction, wherein substantial activation is measurable based on the ability of platelets in the PRP fraction to aggregate to at least one of Ristocetin and Collagen;
hardening the separator substance, via exposure to a UV energy for a period of less than ten minutes, to form a solid barrier, wherein the polymerizable separator substance of the solid barrier has a second average molecular weight that is greater than the first average molecular weight, wherein the solid barrier, after exposure to the UV energy, is stationary with respect to the collection tube and at an intermediate position below the PRP fraction and above the non-PRP fraction that allows removal of at least 95% of the PRP fraction from the collection tube; and
drawing up the PRP fraction, in a closed process that maintains sterility of the PRP fraction, from the collection tube to a fluid dispenser using a transfer needle that contacts the PRP fraction and a first end of the fluid dispenser simultaneously, wherein the fluid dispenser comprises a second end distinct from the first end, the second end configured to dispense the PRP fraction.

2. The method of claim 1, wherein the solid barrier further allows removal of 100% of the PRP fraction from the collection tube.

3. The method of claim 1, further comprising at least one of stirring and agitating the PRP fraction in the collection tube at least one day after the step of hardening the separator substance, without dislodging the solid barrier.

4. The method of claim 1, wherein centrifuging comprises centrifuging the sample of whole blood in the collection tube including the polymerizable separator substance for between 5 and 20 minutes at between 700 and 3600RPM.

5. The method of claim 1, wherein centrifuging comprises centrifuging the sample of whole blood in the collection tube including the polymerizable separator substance at room temperature.

6. The method of claim 5, wherein the separator substance further comprises an antioxidant.

7. The method of claim 1, wherein the photoinitiator is present at a concentration of less than 5%, and wherein the stabilizer is present at a concentration of less than .5 wt%.

8. The method of claim 1, wherein the fluid dispenser is an eye dropper configured to dispense the PRP fraction in a sterile, controlled, drop-wise manner.

## Patentansprüche

1. Verfahren zum Trennen einer PRP-Fraktion aus einer Vollblutprobe in einer Entnahmeröhre, darin eingeschlossen eine polymerisierbare Trennsubstanz, wobei die polymerisierbare Trennsubstanz mit dem Vollblut fließen kann, ein erstes durchschnittliches Molekulargewicht aufweist und eine Dichte zwischen einer durchschnittlichen Dichte einer PRP-Fraktion und eine durchschnittlichen Dichte einer Nicht-PRP-Fraktion aufweist, wobei die Trennsubstanz ein Oligomer, einen Photoinitiator und ein Stabilisierungsmittel umfasst, wobei das Verfahren umfasst:
Zentrifugieren der Vollblutprobe in der Entnahmeröhre, darin eingeschlossen die polymerisierbare Trennsubstanz, für einen Zeitraum zwischen einer und dreißig Minuten bei zwischen 500 und 5000 U/min;
wobei das Zentrifugieren bewirkt, dass die polymerisierbare Trennsubstanz zwischen der PRP-Fraktion und der Nicht-PRP-Fraktion ohne eine wesentliche Aktivierung von Plättchen der PRP-Fraktion fließt, wobei die wesentliche Aktivierung, basierend auf der Fähigkeit von Plättchen in der PRP-Fraktion, an mindestens eines von Ristocetin und Collagen zu aggregieren, gemessen werden kann;
Härten der Trennsubstanz über die Einwirkung einer UV-Energie für einen Zeitraum von weniger als zehn Minuten, um eine feste Barriere zu bilden, wobei die polymerisierbare Trennsubstanz der festen Barriere ein zweites durchschnittliches Molekulargewicht aufweist, das größer als das erste durchschnittliche Molekulargewicht ist, wobei die feste Barriere, nach der Einwirkung der UV-Energie in Bezug auf die Entnahmeröhre stationär ist und sich auf einer Zwischenposition unter der PRP-Fraktion und über der Nicht-PRP Fraktion befindet, die die Entfernung von mindestens 95 % der PRP-Fraktion aus der Entnahmeröhre ermöglicht; und
Abziehen der PRP-Fraktion in einem geschlossenen Prozess, der die Sterilität der PRP-Fraktion beibehält, aus der Entnahmeröhre in eine Fluidabgabevorrichtung unter Verwendung einer Übertragungsnadel, die die PRP-Fraktion und ein erstes Ende der Fluidabgabevorrichtung gleichzeitig berührt, wobei die Fluidabgabevorrichtung ein zweites Ende umfasst, das verschieden vom ersten Ende ist, wobei das zweite Ende konfiguriert ist, um die PRP-Fraktion abzugeben.

2. Verfahren nach Anspruch 1, wobei die feste Barriere außerdem die Entfernung von 100% der PRP-Fraktion aus der Entnahmeröhre ermöglicht.

3. Verfahren nach Anspruch 1, ferner umfassend mindestens eines von Rühren und Schütteln der PRP-Fraktion in der Entnahmeröhre mindestens einen Tag nach dem Schritt des Härtens der Trennsubstanz, ohne die feste Barriere zu verlagern.

4. Verfahren nach Anspruch 1, wobei das Zentrifugieren ein Zentrifugieren der Vollblutprobe in der Entnahmeröhre, darin eingeschlossen die polymerisierbare Trennsubstanz, für zwischen 5 und 20 Minuten bei zwischen 700 und 3600 U/min einschließt.

5. Verfahren nach Anspruch 1, wobei das Zentrifugieren ein Zentrifugieren der Vollblutprobe in der Entnahmeröhre, darin eingeschlossen die polymerisierbare Trennsubstanz bei Raumtemperatur, umfasst.

6. Verfahren nach Anspruch 5, wobei die Trennsubstanz ferner ein Antioxidationsmittel umfasst.

7. Verfahren nach Anspruch 1, wobei der Photoinitiator in einer Konzentration von weniger als 5% vorhanden ist, und wobei das Stabilisierungsmittel in einer Konzentration von weniger als 0.5 Gew.-% vorhanden ist.

8. Verfahren nach Anspruch 1, wobei die Fluidabgabevorrichtung eine Augentropfenpipette ist, die konfiguriert ist, um die PRP-Fraktion auf eine sterile, kontrollierte, tropfenartige Weise abzugeben.

## Revendications

1. Procédé de séparation d'une fraction de PRP d'un échantillon de sang total dans un tube collecteur comprenant une substance séparatrice polymérisable, la substance séparatrice polymérisable étant fluide avec le sang total, ayant une première masse moléculaire moyenne et ayant une densité comprise entre une densité moyenne d'une fraction de PRP et une densité moyenne d'une fraction autre que PRP, la substance séparatrice comprenant un oligomère, un photoamorceur et un stabilisant, le procédé comprenant :
la centrifugation de l'échantillon de sang total dans le tube collecteur comprenant la substance séparatrice polymérisable, pendant une durée comprise entre une et trente minutes à entre 500 et 5 000 tr/minute ;
la centrifugation provoquant l'écoulement de la substance séparatrice polymérisable entre la fraction de PRP et la fraction autre que PRP sans activation substantielle des plaquettes dans la fraction de PRP, une activation substantielle étant mesurable en se basant sur la capacité des plaquettes dans la fraction de PRP à s'agréger à au moins une molécule parmi la ristocétine et le collagène ;
le durcissement de la substance séparatrice, par exposition à une énergie UV pendant une période inférieure à dix minutes, pour former une barrière solide, la substance séparatrice polymérisable de la barrière solide ayant une seconde masse moléculaire moyenne qui est supérieure à la première masse moléculaire moyenne, la barrière solide, après exposition à l'énergie UV, étant stationnaire par rapport au tube collecteur et à une position intermédiaire au-dessous de la fraction de PRP et au-dessus de la fraction autre que PRP, ce qui permet l'élimination d'au moins 95 % de la fraction de PRP du tube collecteur ; et
l'aspiration de la fraction de PRP, dans un procédé fermé qui maintient la stérilité de la fraction de PRP, depuis le tube collecteur vers un distributeur de fluide au moyen d'une aiguille de transfert qui entre en contact simultanément avec la fraction de PRP et une première extrémité du distributeur de fluide, le distributeur de fluide comprenant une seconde extrémité distincte de la première extrémité, la seconde extrémité étant configurée pour distribuer la fraction de PRP.

2. Procédé selon la revendication 1, dans lequel la barrière solide permet en outre l'élimination de 100 % de la fraction de PRP du tube collecteur.

3. Procédé selon la revendication 1, comprenant en outre au moins une étape parmi le mélange et l'agitation de la fraction de PRP dans le tube collecteur au moins un jour après l'étape de durcissement de la substance séparatrice, sans déloger la barrière solide.

4. Procédé selon la revendication 1, dans lequel la centrifugation comprend la centrifugation de l'échantillon de sang total dans le tube collecteur comprenant la substance séparatrice polymérisable pendant entre 5 et 20 minutes à entre 700 et 3600 tr/min.

5. Procédé selon la revendication 1, dans lequel la centrifugation comprend la centrifugation de l'échantillon de sang total dans le tube collecteur comprenant la substance séparatrice polymérisable à température ambiante.

6. Procédé selon la revendication 5, dans lequel la substance séparatrice comprend en outre un antioxydant.

7. Procédé selon la revendication 1, dans lequel le photoamorceur est présent à une concentration inférieure à 5 % et dans lequel le stabilisant est présent à une concentration inférieure à .5 % en poids.

8. Procédé selon la revendication 1, dans lequel le distributeur de fluide est un compte-goutte oculaire configuré pour distribuer la fraction de PRP de manière stérile, contrôlée et au goutte-à-goutte.
